# EUROPÄISCHE PATENTSCHRIFT

(11) **EP 0 621 262 B1**
(45) Veröffentlichungstag und Bekanntmachung des Hinweises auf die Patenterteilung: **11.09.1996**
(21) Anmeldenummer: 94106048.5
(22) Anmeldetag: 19.04.1994
(51) Int. Cl.: C07C 227/18, C07C 229/56, C07D 265/26

(54) **Verfahren zur Herstellung von 3-Chloranthranilsäurealkylestern hoher Reinheit aus 3-Chloranthranilsäure**
Process for the preparation of esters of 3-chloroanthranilic acid of high purity from 3-chloroanthranilic acid
Procédé de préparation d'esters très purs de l'acide 3-chloroanthranilique au départ de cet acide

(30) Priorität: 22.04.1993 DE 4313174
(43) Veröffentlichungstag der Anmeldung: 26.10.1994
(73) Patentinhaber: HOECHST AKTIENGESELLSCHAFT, 65929 Frankfurt am Main (DE)
(72) Erfinder: Semler, Günther, Dr., D-65779 Kelkheim (DE); Meier, Michael, Dr., D-60487 Frankfurt am Main (DE)

(56) Entgegenhaltungen:
- EP-A- 0 020 969
- DE-A- 2 925 175
- DE-A- 3 106 111
- GB-A- 1 549 297
- US-A- 5 068 392
- US-A- 5 118 832
- DEUTSCHE CHEMISCHE GESELLSCHAFT CHEMISCHE BERICHTE Bd. 32 , 1898 Seiten 2159 - 2172 ERNST ERDMANN 'Constitution und Verhalten der "Isatosäure"'

## Beschreibung

Die Erfindung betrifft ein Verfahren zur Herstellung von 3-Chloranthranilsäurealkylestern hoher Reinheit durch Umsetzung von 3-Chloranthranilsäure mit Phosgen in einem inerten organischen Lösungsmittel und Veresterung des so gebildeten 3-Chlorisatosäureanhydrids mit einem Alkanol.

3-Chloranthranilsäurealkylester sind wichtige Zwischenprodukte zur Herstellung von Pharma- und Agroprodukten (siehe z.B.: DE 2 812 586, DE 3 142 727). In Chem. Ber. 32, 2159-2172 (1898) wird die Herstellung von Isatosäure-anhydrid in wäßriger Lösung durch Umsetzung des Natriumsalzes von Anthranilsäure mit Phosgen beschrieben. Die Umsetzung des Natriumsalzes von Anthranilsäure mit Phosgen in Benzol führt zu einem Gemisch aus Anthranilsäure-Hydrochlorid und Isatosäureanhydrid. In der DE 2 925 175 wird die Herstellung von substituierten Isatosäureanhydriden durch Umsetzung der entsprechend Isatine mit Wasserstoffperoxid beschrieben. In GB 1 549 297 ist die Umsetzung von Isatosäureanhydrid mit Alkoholen in Gegenwart von basischen Substanzen bei 70 - 90°C beschrieben.
Die Herstellung von reinen 3-Chloranthranilsäurealkylestern erfolgte bisher nur in einer 5 stufigen Synthese ausgehend von Anthranilsäureestern (US 5 068 392). Gemäß US 5 068 392 lassen sich (reine) 3-Chloranthranilsäurealkylester ausgehend von Anthranilsäureestern mittels einer 5-stufigen Synthese herstellen. Zunächst wird die Aminogruppe des Anthranilsäurealkylesters acetyliert, anschließend in para Stellung zur acetylierten Aminogruppe bromiert, woran sich eine Chlorierung in der ortho-Stellung zur acetylierten Aminogruppe anschließt. Nachfolgend wird mittels einer Reduktion das Brom entfernt und die Acetaminogruppe entacetyliert. Das Verfahren ist recht aufwendig und erweist sich wegen der Verwendung von Bromchlorid oder elementarem Brom und Chlor als problematisch. Zudem entsteht zwangsweise eine Reihe von unverwünschten Abfallprodukten.

Die Darstellung von 3-Chloranthranilsäurealkylestern wurde sonst nur noch als Gemisch mit in 5 bzw. 6 Position substituierten Chloranthranilsäurealkylestern beschrieben.
So wird nach der EP 20 969 3-Chlorphthalsäureanhydrid mit Ammoniak, Alkalihydroxid und Alkalihypochlorit umgesetzt und das erhaltene Gemisch aus 5-Chlor- und 8-Chlor-isatosäureanhydrid mit Alkanolen zu einem Gemisch von 3-Chlor- und 6-Chloranthranilsäurealkylestern verestert.

Bei der Herstellung von 3-Chloranthranilsäurealkylestern gemäß US 5 118 832 durch Umsetzung von Anthranilsäureestern mit 1,3-Dichloro-5,5-dimethyl-hydantoin entstehen 5-Chlor- und 3,5-Dichloranthranilsäurealkylester zu 23 bis 48 % bzw. 2 bis 6 % als Nebenprodukte. 3-Chloranthranilsäuremethylester wird nach aufwendiger Abtrennung der Nebenprodukte in 49,6 % Ausbeute erhalten. Es besteht daher ein Bedarf nach einem einfachen und technisch leicht durchführbaren Verfahren zur Herstellung von 3-Chloranthranilsäurealkylestern, das die vorstehend genannten Nachteile vermeidet und 3-Chloranthranilsäurealkylester nicht nur in hoher Reinheit sondern auch in hoher Ausbeute zugänglich macht.

Diese Aufgabe wird gelöst durch ein Verfahren zur Herstellung von 3-Chloranthranilsäurealkylestern. Es ist dadurch gekennzeichnet, daß man 3-Chloranthranilsäure (1) in einem inerten organischen Lösungsmittel mit 0,8 bis 5 Gewichtsteilen Phosgen und das gebildete 3-Chlorisatosäureanhydrid (2) mit einem Alkanol, gegebenenfalls in Anwesenheit eines Veresterungskatalysators, zu dem 3-Chloranthranilsäurealkylester (3) umsetzt. 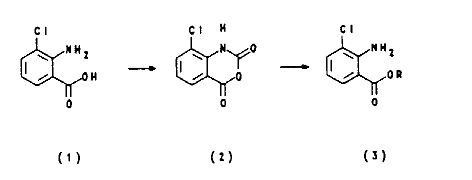 Bei dem erfindungsgemäßen Verfahren wird als inertes organisches Lösungsmittel ein aromatischer Kohlenwasserstoff, ein chlorierter aromatischer Kohlenwasserstoff oder Mischungen derselben eingesetzt. In vielen Fällen hat es sich bewährt Toluol, ein Xylol, ein Gemisch isomerer Xylole, Chlorbenzol, Dichlorbenzol und/oder ein Chlortoluol in etwa 4 bis 20, bevorzugt 8 bis 12 Gewichtsteilen je Gewichtsteil 3-Chloranthranilsäure zu verwenden. An Stelle des reinen Lösungsmittels kann bei Einsatz einer reinen 3-Chloranthranilsäure die Mutterlauge aus der Phosgenierungsstufe eingesetzt werden. Selbstverständlich lassen sich auch größere Mengen Lösungsmittel verwenden, was jedoch die Raumzeitausbeute herabsetzt.

Es hat sich als vorteilhaft erwiesen, in die Reaktion je Gewichtsteil 3-Chloranthranilsäure 0,8 bis 5, insbesondere 1,6 bis 3,1 Gewichtsteile Phosgen einzusetzen.

In vielen Fällen hat es sich bewährt, daß man in einem ersten Schritt 3-Chloranthranilsäure bei Temperaturen von 5 bis 50°C, insbesondere 15 bis 35°C mit 0,5 bis 3, insbesondere 1 bis 2 Gewichtsteilen Phosgen je Gewichtsteil 3-Chloranthranilsäure behandelt, anschließend in einem zweiten Schritt auf Temperaturen von 80 bis 140, insbesondere 90 bis 120°C erwärmt und mit weiteren 0,3 bis 2, insbesondere 0,6 bis 1,1 Gewichtsteilen Phosgen je Gewichtsteil 3-Chloranthranilsäure behandelt, noch vorhandenes Phosgen sorgfältig abgetrennt und das so hergestellte 3-Chlorisatosäureanhydrid durch Filtration isoliert.

Es ist allerdings auch möglich, die Umsetzung von 3-Chloranthranilsäure mit Phosgen in einem einzigen Schritt durchzuführen. Hierbei empfiehlt es sich, bei Temperaturen von 80 bis 120, insbesondere 90 bis 110°C zu arbeiten und das Phosgen in etwa, wie es durch die Reaktion verbraucht wird, zuzusetzen. Für gute Durchmischung ist zu sorgen. Die Phosgenmenge entspricht den für die zweistufige Arbeitsweise angegebenen Bedarf. Erforderlichenfalls läßt man die Umsetzung unter erhöhtem Druck ablaufen, um die Reaktionsdauer niedrig zu halten. Nach Abschluß der Umsetzung ist noch vorhandenes Phosgen gründlich zu entfernen. Das gebildete 3-Chlorisatosäureanhydrid wird anschließend mittels Filtration abgetrennt.

Für die anschließende Umsetzung (Veresterung) des 3-Chlorisatosäureanhydrids können zweckmäßig Alkanole mit 1 bis 4 Kohlenstoffatomen eingesetzt werden.

Als Alkanole lassen sich mit gutem Erfolg Methanol, Ethanol, n-Propanol, Isopropanol, Butanol, Isobutanol, sek.-Butanol und tert.-Butanol, insbesondere Methanol verwenden.

Es hat sich weiterhin als nützlich erwiesen, die Veresterung mit 1 bis 20, insbesondere 2 bis 10 Gewichtsteilen Alkanol pro Gewichtsanteil 3-Chlorisatosäureanhydrid durchzuführen.

Weiterhin hat es sich bewährt die Veresterung bei 5 bis 90°C, insbesondere bei 15 bis 75°C durchzuführen. Bei höheren Temperaturen und zu langen Reaktionszeiten können in Abhängigkeit von den Katalysatoren Ausbeuteverluste auftreten.

Als Veresterungskatalysatoren können die in der Literatur beschriebenen Verbindungen verwendet werden (siehe z.B. EP 20 969). Geeignet sind basische Verbindungen, wie die Hydroxide, Alkoholate, Carbonate und/oder Hydrogencarbonate der Alkalimetalle, insbesondere die entsprechenden Verbindungen des Natriums und/oder Kaliums.

Der Katälysator wird üblicherweise in 0,01 bis 0,8, insbesondere 0,1 bis 0,5 Äquivalenten je Äquivalent 3-Chlorisatosäureanhydrid eingesetzt.

Ein zusätzlicher Vorteil des erfindungsgemäßen Verfahrens ist, daß auch 3-Chloranthranilsäure mit technischer Qualität (Reinheit etwa 90 %) eingesetzt werden kann, und trotzdem 3-Chloranthranilsäurealkylester von hoher Reinheit gewonnen wird.Als Einsatzprodukt eignet sich zum Beispiel eine 3-Chloranthranisäure, die nach dem Verfahren der EP 0 528 375 erhalten wird.

Nach dem oben beschriebenen Verfahren werden 3-Chloranthranilsäurealkylester in ca. 90 % Ausbeute bezogen auf 3-Chloranthranilsäure gebildet. Die Reinheit ist > 99 %.

Die nachstehenden Beispiele dienen zur Erläuterung des erfindungsgemäßen Verfahrens, ohne es zu beschränken.

### Beispiel 1

### Herstellung von 3-Chlorisatosäureanhydrid

In einem Vierhalskolben mit Rührer, Thermometer und einem auf -20 °C gekühlten Rückflußkühler werden 190,7 g 3-Chloranthranilsäure 90 %ig, entsprechend 171,6 g (1,0 mol) 3-Chloranthranilsäure 100 %ig, in 1970,0 g Chlorbenzol vorgelegt und unter gutem Rühren 300,0 g Phosgen in ca. 3 h bei Raumtemperatur eindosiert. Das Reaktionsgemisch wird auf 100°C erwärmt und es werden in 2 h nochmals 150,0 g Phosgen eingeleitet. Danach wird 1 h bei 100°C nachgerührt und anschließend bei dieser Temperatur mit Stickstoff das Phosgen entfernt. Nach Abkühlen auf ca. 25°C, wird das ausgefallene Kristallisat abgesaugt, der Filterkuchen mit 2 x 50 ml Chlorbenzol gewaschen und im Vakuum bei 100°C getrocknet. Man erhält 190,0 g 3-Chlorisatosäureanhydrid, mit einer Reinheit nach GC von > 99 %, dies entspricht 96,2 % d. Th.
Nachfolgend steht Schmp. für Schmelzpunkt, EP für Erstarrungspunkt, MS für Massenspektrum.
Schmp.: 223°C
MS: 197 (M⁺), 153 (M±-CO₂), 125

### Beispiel 2

### Herstellung von 3-Chlorisatosäureanhydrid

In einem Vierhalskolben mit Rührer, Thermometer und auf -20°C gekühlten Rückflußkühler werden 190,7 g 3-Chloranthranilsäure 90 %ig, entsprechend 171,6 g (1,0 mol) 3-Chloranthranilsäure 100 %ig in 1820,0 g Toluol vorgelegt und unter guten Rühren 300,0 g Phosgen in ca. 3 h bei Raumtemperatur eindosiert. Das Reaktionsgemisch wird auf 100°C erwärmt, und in 2 h nochmals 150,0 g Phosgen eingeleitet. Danach wird 1 h bei 100°C nachgerührt und anschließend bei dieser Temperatur mit Stickstoff phosgenfrei geblasen. Nach Abkühlen auf ca. 25°C, wird das ausgefallene Kristallisat abgesaugt, der Filterkuchen mit 2 x 50 ml Toluol gewaschen und im Vakuum bei 100°C getrocknet. Man erhält 188,3 g 3-Chlorisatosäureanhydrid, mit einer Reinheit nach GC von > 99 %, dies entspricht 95,2 % d.Th.
Schmp.: 223°C
MS: 197 (M⁺), 153 (M⁺-CO₂), 125

### Beispiel 3

### Herstellung von 3-Chlorisatosäureanhydrid (einstufig)

In einem Vierhalskolben mit Rührer, Thermometer und einem bei -20°C betriebenen Rückflußkühler werden 171,6 g (1,0 mol) 3-Chloranthranilsäure 99,7 %ig in 1720,0 g Chlorbenzol vorgelegt und unter gutem Rühren 440,0 g Phosgen in ca. 3 h bei 100°C eingegast. Danach wird 1 h bei 100°C nachgerührt und anschließend bei dieser Temperatur mit Stickstoff phosgenfrei geblasen. Nach Abkühlen auf ca. 25°C, wird das ausgefallene Kristallisat abgesaugt, der Filterkuchen mit 2 x 50 ml Chlorbenzol gewaschen und im Vakuum bei 100°C getrockent. Man erhält 184,9 g 3-Chlorisatosäureanhydrid, mit einer Reinheit nach GC von > 99 %, dies entspricht 93,6 % d. Th.
Schmp.: 223°C.
MS: 197 (M⁺), 153 (M⁺-CO₂), 125

### Beispiel 4

### Herstellung von 3-Chloranthranilsäuremethylester

In 200 g Methanol werden 98,8 g (0,5 mol) 3-Chlorisatosäureanhydrid und 25 g Natriumcarbonat bei 25°C eingerührt und 3 h bei dieser Temperatur nachgerührt. Anschließend wird filtriert, mit Methanol gewaschen und bei Normaldruck das Methanol abdestilliert und dann der 3-Chloranthranilsäuremethylester bei 115°C/15 mbar destilliert. Die Ausbeute beträgt 86,3 g, entsprechend 93,0 % d.Th. Die Reinheit nach GC beträgt > 99,5 %.
EP.: 36,7°C
MS: 185 (M⁺), 153 (M⁺-CH₃OH), 125

### Beispiel 5

### Herstellung von 3-Chloranthranilsäuremethylester

In 250 g Methanol werden 98,8 g (0,5 mol) 3-Chlorisatosäureanhydrid und 9,0 g Kaliumcarbonat bei 25°C eingerührt und 3 h bei dieser Temperatur nachgerührt. Anschließend wird filtriert, mit Methanol gewaschen und bei Normaldruck das Methanol abdestilliert und 87,7 g 3-Chloranthranilsäuremethylester bei 115°C/15 mbar destilliert. Dies entspricht einer Ausbeute von 94,5 % d.Th. Die Reinheit nach GC beträgt > 99,5 %.
EP.: 37,5°C
MS: 185 (M⁺), 153 (M⁺-CH₃OH), 125

### Beispiel 6

### Herstellung von 3-Chloranthranilsäuremethylester

In 200 g Methanol werden 98,8 g (0,5 mol) 3-Chlorisatosäureanhydrid eingerührt, 10 g Natriummethanolat 30 %ig in Methanol bei 25°C zugegeben, auf 68°C erwärmt und 2 h bei dieser Temperatur nachgerührt. Anschließend wird filtriert, mit Methanol gewaschen und bei Normaldruck das Methanol abdestilliert und 84,8 g 3-Chloranthranilsäuremethylester bei 115°C/15 mbar destilliert. Dies entspricht einer Ausbeute von 91,4 % d.Th. Die Reinheit nach GC beträgt > 99,5 %.
EP.: 37,0°C
MS: 185 (M⁺), 153 (M⁺-CH₃OH), 125

## Patentansprüche

1. Verfahren zur Herstellung von 3-Chloranthranilsäurealkylestern, dadurch gekennzeichnet, daß man 3-Chloranthranilsäure in einem inerten Lösungsmittel mit 0,8 bis 5 Gewichtsteilen Phosgen und das gebildete 3-Chlorisatosäureanhydrid mit einem Alkanol gegebenenfalls in Anwesenheit eines Veresterungskatalysators zu dem 3-Chloranthranilsäurealkylester umsetzt.

2. Verfahren nach Anspruch 1, dadurch gekennzeichnet, daß man als inertes Lösungsmittel einen aromatischen Kohlenwasserstoff, einen chlorierten aromatischen Kohlenwasserstoff oder Mischungen derselben einsetzt.

3. Verfahren nach einem oder mehreren der Ansprüche 1 und 2, dadurch gekennzeichnet, daß man als inertes Lösungsmittel Toluol, ein Xylol, ein Gemisch isomerer Xylole, Chlorbenzol, Dichlorbenzol und/oder ein Chlortoluol einsetzt

4. Verfahren nach einem oder mehreren der Ansprüche 1 bis 3; dadurch gekennzeichnet, daß man je Gewichtsteil 3-Chloranthranilsäure 4 bis 20, insbesondere 8 bis 12 Gewichtsteile inertes Lösungsmittel einsetzt.

5. Verfahren nach einem oder mehreren der Ansprüche 1 bis 4, dadurch gekennzeichnet, daß man je Gewichtsteil 3-Chloranthranilsäure 0,8 bis 5, insbesondere 1,6 bis 3,1 Gewichtsteile Phosgen einsetzt.

6. Verfahren nach einem oder mehreren der Ansprüche 1 bis 5, dadurch gekennzeichnet, daß man in einem ersten Schritt 3-Chloranthranilsäure mit 0,5 bis 3 Gewichtsteiten Phosgen je Gewichtsteil 3-Chloranthranisäure umsetzt.

7. Verfahren nach einem oder mehreren der Ansprüche 1 bis 6, dadurch gekennzeichnet, daß man im ersten Schritt 3-Chloranthranilsäure mit 1 bis 2 Gewichtsteilen Phosgen je Gewichtsteil 3-Chloranthranilsäure umsetzt.

8. Verfahren nach einem oder mehreren der Ansprüche 1 bis 7, dadurch gekennzeichnet, daß man im ersten Schritt 3-Chloranthranilsäure mit Phosgen bei 5 bis 50°C umsetzt.

9. Verfahren nach einem oder mehreren der Ansprüche 1 bis 8, dadurch gekennzeichnet, daß man im ersten Schritt 3-Chloranthranilsäure mit Phosgen bei 15 bis 35°C umsetzt.

10. Verfahren nach einem oder mehreren der Ansprüche 1 bis 9, dadurch gekennzeichnet, daß man in einem zweiten Schritt das aus dem ersten Schritt resultierende Reaktionsprodukt mit 0,3 bis 2 Gewichtsteilen Phosgen je Gewichtsteil 3-Chloranthranilsäure umsetzt.

11. Verfahren nach einem oder mehreren der Ansprüche 1 bis 10, dadurch gekennzeichnet, daß man im zweiten Schritt das Reaktionsprodukt mit 0,6 bis 1,1 Gewichtsteilen Phosgen je Gewichtsteil 3-Chloranthranilsäure umsetzt.

12. Verfahren nach einem oder mehreren der Ansprüche 1 bis 11, dadurch gekennzeichnet, daß man im zweiten Schritt das Reaktionsprodukt mit Phosgen bei 80 bis 140°C umsetzt.

13. Verfahren nach einem oder mehreren der Ansprüche 1 bis 12, dadurch gekennzeichnet, daß man im zweiten Schritt das Reaktionsprodukt mit Phosgen bei 90 bis 120°C umsetzt.

14. Verfahren nach einem oder mehreren der Ansprüche 1 bis 5, dadurch gekennzeichnet, daß man 3-Chloranthranilsäure mit Phosgen in einem einzigen Schritt bei 80 bis 120, insbesondere 90 bis 110°C umsetzt.

15. Verfahren nach einem oder mehreren der Ansprüche 1 bis 14, dadurch gekennzeichnet, daß man das 3-Chlorisatosäureanhydrid mit einem Alkanol mit 1 bis 4 Kohlenstoffatomen umsetzt.

16. Verfahren nach einem oder mehreren der Ansprüche 1 bis 15, dadurch gekennzeichnet, daß man je Gewichtsteil 3-Chlorisatosäureanhydrid 1 bis 20, insbesondere 2 bis 10 Gewichtsteile Alkanol einsetzt.

17. Verfahren nach einem oder mehreren der Ansprüche 1 bis 16, dadurch gekennzeichnet, daß man das 3-Chlorisatosäureanhydrid mit dem Alkanol bei 5 bis 90°C umsetzt.

18. Verfahren nach einem oder mehreren der Ansprüche 1 bis 17, dadurch gekennzeichnet, daß man das 3-Chlorisatosäureanhydrid mit dem Alkanol bei 15 bis 75°C umsetzt.

19. Verfahren nach einem oder mehreren der Ansprüche 1 bis 18, dadurch gekennzeichnet, daß man als Veresterungskatalysator eine basische Verbindung, insbesondere ein Hydroxid, Carbonat und/oder Hydrogencarbonat eines Alkalimetalls vorzugsweise des Natriums und/oder Kaliums einsetzt.

20. Verfahren nach einem oder mehreren der Ansprüche 1 bis 19, dadurch gekennzeichnet, daß man je Äquivalent 3-Chlorisatosäureanhydrid 0,01 bis 0,8, insbesondere 0,1 bis 0,5 Äquivalent Veresterungkatalysator einsetzt.

## Claims

1. A process for preparing 3-chloroanthranilic alkyl esters, which comprises reacting 3-chloroanthranilic acid, in an inert solvent, with from 0.8 to 5 parts by weight of phosgene, and reacting the 3-chloroisatoic anhydride which is formed with an alkanol, where appropriate in the presence of an esterification catalyst, to give the 3-chloroanthranilic alkyl ester.

2. The process as claimed in claim 1, wherein an aromatic hydrocarbon, a chlorinated aromatic hydrocarbon, or mixtures thereof, is/are employed as the inert solvent.

3. The process as claimed in one or more of claims 1 and 2, wherein toluene, a xylene, a mixture of xylene isomers, chlorobenzene, dichlorobenzene and/or a chlorotoluene is employed as the inert solvent.

4. The process as claimed in one or more of claims 1 to 3, wherein from 4 to 20, in particular from 8 to 12, parts by weight of inert solvent are employed for each part by weight of 3-chloroanthranilic acid.

5. The process as claimed in one or more of claims 1 to 4, wherein from 0.8 to 5, in particular from 1.6 to 3.1, parts by weight of phosgene are employed for each part by weight of 3-chloroanthranilic acid.

6. The process as claimed in one or more of claims 1 to 5, wherein 3-chloroanthranilic acid is reacted, in a first step, with from 0.5 to 3 parts by weight of phosgene for each part by weight of 3-chloroanthranilic acid.

7. The process as claimed in one or more of claims 1 to 6, wherein 3-chloroanthranilic acid is reacted, in the first step, with from 1 to 2 parts by weight of phosgene for each part by weight of 3-chloroanthranilic acid.

8. The process as claimed in one or more of claims 1 to 7, wherein 3-chloroanthranilic acid is reacted, in the first step, with phosgene at from 5 to 50°C.

9. The process as claimed in one or more of claims 1 to 8, wherein 3-chloroanthranilic acid is reacted, in the first step, with phosgene at from 15 to 35°C.

10. The process as claimed in one or more of claims 1 to 9, wherein the reaction product resulting from the first step is reacted, in a second step, with from 0.3 to 2 parts by weight of phosgene for each part by weight of 3-chloroanthranilic acid.

11. The process as claimed in one or more of claims 1 to 10, wherein the reaction product is reacted, in the second step, with from 0.6 to 1.1 parts by weight of phosgene for each part by weight of 3-chloroanthranilic acid.

12. The process as claimed in one or more of claims 1 to 11, wherein the reaction product is reacted, in the second step, with phosgene at from 80 to 140°C.

13. The process as claimed in one or more of claims 1 to 12, wherein the reaction product is reacted, in the second step, with phosgene at from 90 to 120°C.

14. The process as claimed in one or more of claims 1 to 5, wherein 3-chloroanthranilic acid is reacted with phosgene in a single step at from 80 to 120, in particular from 90 to 110, °C.

15. The process as claimed in one or more of claims 1 to 14, wherein the 3-chloroisatoic anhydride is reacted with an alkanol having 1 to 4 carbon atoms.

16. The process as claimed in one or more of claims 1 to 15, wherein from 1 to 20, in particular from 2 to 10, parts by weight of alkanol are employed for each part by weight of 3-chloroisatoic anhydride.

17. The process as claimed in one or more of claims 1 to 16, wherein the 3-chloroisatoic anhydride is reacted with the alkanol at from 5 to 90°C.

18. The process as claimed in one or more of claims 1 to 17, wherein the 3-chloroisatoic anhydride is reacted with the alkanol at from 15 to 75°C.

19. The process as claimed in one or more of claims 1 to 18, wherein a basic compound, in particular a hydroxide, carbonate and/or hydrogen carbonate of an alkali metal, preferably of sodium and/or potassium, is employed as the esterification catalyst.

20. The process as claimed in one or more of claims 1 to 19, wherein from 0.01 to 0.8, in particular from 0.1 to 0.5, of an equivalent of esterification catalyst is employed for each equivalent of 3-chloroisatoic anhydride.

## Revendications

1. Procédé de préparation d'esters alkyliques de l'acide 3-chloroanthranilique, caractérisé en ce que l'on fait réagir de l'acide 3-chloroanthranilique dans un solvant inerte avec 0,8 à 5 parties en masse de phosgène et en ce que l'on fait réagir l'anhydride d'acide 3-chlorisatoïque formé avec un alcanol, éventuellement en présence d'un catalyseur d'estérification, pour former l'ester alkylique d'acide 3-chloroanthranilique.

2. Procédé selon la revendication 1, caractérisé en ce que l'on utilise comme solvant inerte un hydrocarbure aromatique, un hydrocarbure aromatique chloré ou leurs mélanges.

3. Procédé selon l'une ou plusieurs des revendications 1 et 2, caractérisé en ce que l'on utilise comme solvant inerte du toluène, un xylène, un mélange de xylènes isomères, du chlorobenzène, un dichlorobenzène et/ou un chlorotoluène.

4. Procédé selon l'une ou plusieurs des revendications 1 à 3, caractérisé en ce que l'on utilise environ 4 à 20, en particulier 8 à 12 parties en masse de solvant inerte par partie en masse d'acide 3-chloroanthranilique.

5. Procédé selon l'une ou plusieurs des revendications 1 à 4, caractérisé en ce que l'on utilise 0,8 à 5, en particulier 1,6 à 3,1 parties en masse de phosgène par partie en masse d'acide 3-chloroanthranilique.

6. Procédé selon l'une ou plusieurs des revendications 1 à 5, caractérisé en ce que l'on fait réagir dans une première étape l'acide 3-chloroanthranilique avec 0,5 à 3 parties en masse de phosgène par partie en masse d'acide 3-chloroanthranilique.

7. Procédé selon l'une ou plusieurs des revendications 1 à 6, caractérisé en ce que l'on fait réagir dans la première étape l'acide 3-chloroanthranilique avec 1 à 2 parties en masse de phosgène par partie en masse d'acide 3-chloroanthranilique.

8. Procédé selon l'une ou plusieurs des revendications 1 à 7, caractérisé en ce que l'on fait réagir dans la première étape l'acide 3-chloroanthranilique avec le phosgène à une température de 5 à 50°C.

9. Procédé selon l'une ou plusieurs des revendications 1 à 8, caractérisé en ce que l'on fait réagir dans la première étape l'acide 3-chloroanthranilique avec le phosgène à une température de 15 à 35°C.

10. Procédé selon l'une ou plusieurs des revendications 1 à 9, caractérisé en ce que, dans une deuxième étape, on fait réagir le produit réactionnel résultant de la première étape avec 0,3 à 2 parties en masse de phosgène par partie en masse d'acide 3-chloroanthranilique.

11. Procédé selon l'une ou plusieurs des revendications 1 à 10, caractérisé en ce que, dans la deuxième étape, on fait réagir le produit réactionnel avec 0,6 à 1,1 partie en masse de phosgène par partie en masse d'acide 3-chloroanthranilique.

12. Procédé selon l'une ou plusieurs des revendications 1 à 11, caractérisé en ce que, dans la deuxième étape, on fait réagir le produit réactionnel avec du phosgène à une température de 80 à 140°C.

13. Procédé selon l'une ou plusieurs des revendications 1 à 12, caractérisé en ce que, dans la deuxième étape, on fait réagir le produit réactionnel avec du phosgène à une température de 90 à 120°C.

14. Procédé selon l'une ou plusieurs des revendications 1 à 5, caractérisé en ce que l'on fait réagir l'acide 3-chloroanthranilique avec le phosgène en une seule étape à une température de 80 à 120°C, en particulier de 90 à 110°C.

15. Procédé selon l'une ou plusieurs des revendications 1 à 14, caractérisé en ce que l'on fait réagir l'anhydride d'acide 3-chlorisatoïque avec un alcanol de 1 à 4 atomes de carbone.

16. Procédé selon l'une ou plusieurs des revendications 1 à 15, caractérisé en ce que l'on utilise 1 à 20, en particulier 2 à 10 parties en masse d'alcanol par partie en masse d'anhydride d'acide 3-chlorisatoïque.

17. Procédé selon l'une ou plusieurs des revendications 1 à 16, caractérisé en ce que l'on fait réagir l'anhydride d'acide 3-chlorisatoïque avec l'alcanol à une température de 5 à 90°C.

18. Procédé selon l'une ou plusieurs des revendications 1 à 17, caractérisé en ce que l'on fait réagir l'anhydride d'acide 3-chlorisatoïque avec l'alcanol à une température de 15 à 75°C.

19. Procédé selon l'une ou plusieurs des revendications 1 à 18, caractérisé en ce que l'on utilise comme catalyseur d'estérification un composé basique, en particulier un hydroxyde, un carbonate et/ou un hydrogénocarbonate d'un métal alcalin, de préférence de sodium ou de potassium.

20. Procédé selon l'une ou plusieurs des revendications 1 à 19, caractérisé en ce que l'on utilise 0,01 à 0,8, en particulier 0,1 à 0,5 équivalent de catalyseur d'estérification par équivalent d'anhydride d'acide 3-chlorisatoïque.
